## Europäisches Patentamt
## European Patent Office
## Office européen des brevets

(19)

(11) Numéro de publication: **0 000 463**
**B1**

(12)

# FASCICULE DE BREVET EUROPEEN

(45) Date de publication du fascicule du brevet: **06.01.82**

(51) Int. Cl.³: **C 07 C 118/00,**
**C 07 C 119/048**

(21) Numéro de dépôt: **78400051.5**

(22) Date de dépôt: **03.07.78**

(54) Procédé de séparation du toluène diisocyanate à partir des résidus de fabrication.

(30) Priorité: **12.07.77 FR 7721436**

(43) Date de publication de la demande:
**24.01.79 Bulletin 79/2**

(45) Mention de la délivrance du brevet:
**06.01.82 Bulletin 82/1**

(84) Etats contractants désignés:
**BE DE FR GB LU NL**

(56) Documents cités:
**FR - 2 103 035**
**FR - A - 2 290 419**
**FR - A - 2 320 938**
**US - A - 3 405 040**
**US - A - 3 694 323**

(73) Titulaire: **P C U K PRODUITS CHIMIQUES UGINE KUHLMANN**
**Service Propriété Industrielle Tour Manhattan**
**F-92087 PARIS LA DEFENSE 2 Cédex 21 (FR)**

(72) Inventeur: **Ailloud, Pierre**
**36 Allée Gabriel**
**F-59700 Marcq en Baroeul (FR)**
Inventeur: **D'Haussy Philippe**
**218 rue Sadi Carnot**
**F-59350 Saint Andre (FR)**

(74) Mandataire: **Monceaux, Pierre et al,**
**PRODUITS CHIMIQUES UGINE KUHLMANN**
**Service Propriété Industrielle**
**Tour Manhattan Cédex 21 F-92087 Paris La Defense (FR)**

Courier Press, Leamington Spa, England.

Procédé de séparation du toluène diisocyanate à partir des résidus de fabrication

La présente invention concerne un procédé de séparation de toluène diisocyanate (TDI) des résidus de fabrication.

Lors de la fabrication du TDI il se forme de 10 à 15% de composés lourds liquides. Ces composés lourds sont concentrés par distillation du TDI jusqu'à la concentration type de 30 à 40% de TDI et de 60 à 70% de composés lourds. En raison de l'augmentation de la viscosité du mélange, il est difficile de poursuivre la distillation et d'obtenir des concentrations plus élevées en composés lourds.

En conséquence il faut mettre en oeuvre d'autres techniques pour récupérer le TDI du mélange et recueillir les composés lourds sous forme de résidus combustibles ne contenant plus de TDI, pour éviter à la fois la pollution par des vapeurs toxiques et les pertes de produit onéreuses.

Divers procédés ont été proposés pour cette opération, par exemple celui décrit dans le brevet américain 3.405.040 où le mélange TDI/composés lourds est envoyé dans de l'huile à 220°C sous une pression de 2 666 Pa. Le TDI subit une évaporation flash et les composés lourds précipitent sous forme de résidus solides qui sont séparés de l'huile par essorage puis brûlés.

Un autre procédé décrit dans le brevet français 2.091.813 préconise l'extraction des composés lourds par un solvant, récupéré ensuite par distillation.

Toutefois, ces procédés présentent les inconvénients d'être discontinus et coûteux en matière première et en énergie.

D'autres procédés tentent de supprimer ces inconvénients en proposant la séparation directe du TDI et des composés lourds par évaporation sous vide du TDI et transformation directe des composés lourds en solides.

Toutefois ces procédés présentent les inconvénients d'être soit discontinus, tel le procédé décrit dans le brevet US 3.457.291, soit difficiles à mettre en oeuvre, tel le procédé du BF 2.320.938.

Tous ces procédés de séparation directe ont pour caractéristique d'obtenir très rapidement une couche mince de résidus solides soit sur une paroi chaude (BF 2.320.938), soit sur un lit de résidus solides chauds de façon à éviter les risques de polymérisation du TDI et des composés lourds, sous l'effet de la température et du temps de séjour, et le dégagement de gaz carbonique ou d'autres gaz provenant de la décomposition d'amines réactives avec le TDI, risquant ainsi de former des mousses au sein de la masse. Or, les appareils d'évaporation à couche mince raclée sont chers et d'exploitation très difficile en raison du faible temps de séjour des produits, difficulté signalée dans le BF 2.091.813 déjà cité.

La demanderesse a mis au point un procédé entièrement continu de séparation du TDI à partir des lourds de fabrication, et d'extraction de ces résidus sous forme de solide, qui présente l'avantage d'être simple, d'une utilisation facile et fiable, sans formation de mousse ni produit de décomposition, les résidus ne contenant plus que de faibles teneurs en TDI.

La séparation a lieu dans un évaporateur de type agité et raclé, tel par exemple celui décrit dans le BF 2.039.628.

Les conditions de pression, de température, de temps de séjour ont été étudiées de façon à éviter la décomposition du TDI en gaz et polymères ou produits divers, et la formation de mousse qui rendrait impossible l'utilisation de tels appareils.

Il est prouvé qu'à partir de 220/250°C la décomposition du TDI en $CO_2$ et gaz divers est importante et rapide, et que le dégagement de gaz au sein de la masse visqueuse provoque des mousses. Cependant, le mélange doit être suffisamment chauffé pour que le majeure partie du TDI soit évacuée.

La demanderesse a apporté à ce problème une solution qui consiste à chauffer en continu le mélange TDI/produits lourds sur une paroi à une température maxima de 260°C, à évaporer le TDI à une température débutant de 100 à 130°C sous une pression de 666,5 à 2666 Pa en agitant le mélange pendant un temps suffisant pour favoriser le transfert de masse. Le mélange est ensuite progressivement porté tout au long de l'appareil jusqu'à une température de 220 à 260°C environ, pendant qu'il passe de l'état visqueux à l'état pâteux puis à l'état solide.

L'agitation doit être suffisante pour assurer l'échange thermique, la progression des produits dans l'évaporateur, le raclage des solides et le cassage des mousses éventuellement formées.

Le volume de l'appareil doit être tel qu'il permette un temps de séjour suffisant pour assurer l'échange des matières (de 15 à 45 minutes).

Les solides sortant contiennent moins de 3% de TDI. Leur température peut être sans risque de moussage portée à une température de 260°C en fin d'appareil pour évacuer les dernières traces de TDI.

Les résidus secs, poreux et fragmentés s'écoulent aisément, soit dans un système de recette sous vide fonctionnant en sas, ou mieux encore sont extrudés par vis chauffée, de façon continue à travers une filière assurant l'étanchéité au vide.

Les résidus solides sous forme de poudre ou de granulés compacts sont repris par transport pneumatique et dirigés vers un incinérateur, tandis que le TDI est condensé de façon classique.

Le procédé de l'invention présente les avantages de pouvoir fonctionner en continu d'une manière entièrement automatique et de garantir toutes les conditions de non pollution. En outre, il nécessite peu l'énergie thermique ou mécanique et est de ce fait très économique.

Un appareil de 20 à 25 m2 et de 1 600 litres, suivi d'une extrudeuse, est suffisant pour assurer la séparation continue des lourds et du TDI d'une unité de 30.000 tonnes/an de TDI.

Les exemples suivants illustrent l'invention sans toutefois la limiter.

### Exemple 1

Un mélange composé de 43% de TDI et de 57% de composés lourds issu du fond de colonne de concentration est introduit dans l'appareil à raison de 60 kg/h m2. L'appareil a une surface chauffée de 0,86 m2 et un volume total de 20 litres et possède 2 vis d'agitation autonettoyantes tournant à des vitesses différentes. Sous une pression de 1 600 Pa, le mélange est progressivement porté à 215°C, et se transforme en une poudre contenant 2,8% de TDI après un temps de séjour de 15 mn.

Le TDI est condensé et recueilli, et les résidus solides sont extrudés par pression à travers une filière sous forme de granulés de 6 mm de diamètre et de 15 mm de longueur, à un débit de 30 kg/h.

### Exemples 2 à 4

En opérant comme dans l'exemple 1, mais avec des débits d'alimentation et des temps de séjour différents, on a obtenu les résultats suivants:

| Débit d'alimentation (en kg/h m2) | Temps de séjour (en minutes) | Teneur finale en TDI (en % en poids de résidu) |
|---|---|---|
| 50 | 18 | 1,5 |
| 70 | 13 | 3,4 |
| 25 | 36 | 0,05 |

L'exemple n° 3 montre qu'il faut un temps de séjour minimum, que nous avons fixé à 15 mn, si l'on veut obtenir une faible teneur en TDI des résidus.

### Revendications

1. Procédé de séparation sans décomposition, entièrement continu et automatique du toluène diisocyanate à partir des résidus de fabrication, caractérisé par une évaporation du toluène diisocyanate dans un évaporateur agité et raclé, à une température débutant à 100—130°C sous une pression de 666,5 à 2 666 Pa portée ensuite progressivement jusqu'à une température de 220 à 260°C avec un temps de séjour minimum de 15 mn dans l'évaporateur, et par l'extraction en continu des résidus.

2. Procédé selon la revendication 1, où le résidu d'évaporation est évacué par un système d'extrusion.

### Patentansprüche

1. Verfahren zur völlig kontinuierlichen und automatischen Abtrennung ohne Auflösung von Toluoldiisocyanat aus Herstellungsrückständen ohne Zersetzung gekennzeichnet durch eine Verdampfung des Toluoldiisocyanats in einem gerührten und abstreifenden Verdampfer bei einer anfänglichen Temperatur zwischen 100 und 130°C unter einem Druck von 666,5 bis 2666 Pa und anschließende Erwärmung auf eine Temperatur von 220 bis 260°C bei einer Mindestverweilzeit von 15 min in dem Verdampfer und durch kontinuierliche Extraktion der Rückstände.

2. Verfahren gemäß Anspruch 1, bei dem der Verdampfungsrückstand durch ein Extrudersystem ausgetragen wird.

### Claims

1. Process for the entirely continuous and automatic decomposition-free separation of toluene diisocyanate from production residues, characterised by an evaporation of the toluene diisocyanate in an agitated and scraped evaporator, at a temperature starting at 100—130°C, and under a pressure of 666.5 to 2,666 Pa, thereafter progressively brought to a temperature of 220 to 260°C, with a minimum residence time of 15 minutes in the evaporator, and characterised by continuous extraction of the residues.

2. Process according to Claim 1, wherein the evaporation residue is discharged by an extrusion system.